# EUROPEAN PATENT SPECIFICATION

(11) **EP 2 811 968 B1**
(45) Date of publication and mention of the grant of the patent: **31.10.2018**
(21) Application number: 13746918.5
(22) Date of filing: 08.02.2013
(51) Int. Cl.: A61K 8/36, A61K 8/37, A61K 8/39, A61K 8/44, A61K 8/49, A61K 8/60, A61K 8/63, A61Q 5/02, A61Q 19/10, A61K 8/34, A61K 8/02, A61K 8/14

(54) **STRUCTURED SURFACTANT SUSPENDING SYSTEMS**
STRUKTURIERTE TENSIDSUSPENSIONSSYSTEME
SYSTÈMES DE TENSIOACTIFS STRUCTURÉS AYANT LA CAPACITÉ DE MISE EN SUSPENSION

(30) Priority: 10.02.2012 GB 201202333
(43) Date of publication of application: 17.12.2014
(73) Proprietor: Stepan Company, Northfield, Illinois 60093 (US)
(72) Inventor: HAWKINS, John, F-38000 Grenoble (FR); PACE, Emilie, F-38000 Grenoble (FR); Lebert, Laetitia, F-38700 La Tronche (FR)
(74) Representative: Schnappauf, Georg
(86) International application number: PCT/US2013/025282
(87) International publication number: WO 2013/119908

(56) References cited:
- EP-A1- 0 086 614
- EP-A2- 0 820 755
- WO-A1-2009/098469
- WO-A2-2005/053612
- WO-A2-2010/054495
- CA-C- 2 168 260
- GB-A- 2 464 393
- US-A- 5 783 200
- US-A- 5 866 110
- US-A- 5 945 092
- US-A1- 2011 117 019
- US-A1- 2011 223 125
- US-B1- 6 277 404
- BENDEJACQ DENIS ET AL: "Structured Surfactant Systems for High Performance Shampoos", COSMETICS & TOILETRIES, WHEATON, IL, US, vol. 125, no. 11, 1 November 2010 (2010-11-01), pages 22-29, XP008176922, ISSN: 0361-4387

## Description

### FIELD OF THE INVENTION

The present technology relates to structured surfactant suspending systems and compositions containing the structured surfactant suspending systems, particularly personal care compositions.

### BACKGROUND OF THE INVENTION

Formulating suspensions of water insoluble, or sparingly soluble solids and/or liquids in personal care compositions such as shampoos and body washes presents a long-standing problem. Formulators need to be able to suspend a variety of such ingredients. For example oils, anti-dandruff agents, such as zinc pyrithione, hair conditioners including cationic polymers, and opacifiers such as mica are widely used. There is therefore a need to disperse/suspend them in aqueous shampoos and body washes.

We have discovered novel structured surfactant systems that are capable of suspending solid particles and oils without sedimentation, using high foaming surfactant systems which give dense stable foams and good skin feel.

The term "structured system" as used herein means a pourable composition comprising water, surfactant, and optionally other dissolved matter, which together form a mesophase, or a dispersion of a mesophase in a continuous aqueous medium, and which has the ability to immobilize non-colloidal, water-insoluble particles, while the system is at rest, thereby forming a stable, pourable suspension. Surfactants and water interact to form phases that are neither liquids nor crystals; these are usually termed "liquid crystal phases," or alternatively "mesomorphic phases" or "mesophases."

The term "pourable" is used herein to refer to shear thinning fluids having viscosities around 2000 mPa·s (2000 cps) (Brookfield RVT viscometer, spindle 5, speed 100) at room temperature.

Attempts to solve the problem of dispersing water insoluble materials in water have generally involved either using gums or other polymeric thickeners to raise the viscosity of the liquid medium, or else forming colloidal dispersions. More recently the use of lamellar structured surfactants has been proposed.

Gums and polymeric thickeners, which increase the viscosity of the liquid medium, retard, but do not prevent sedimentation, and at the same time make the composition harder to pour. They do not provide stable suspensions.

Colloidal dispersions are prevented from sedimenting by Brownian motion. Such systems are usually incapable of dispersing relatively coarse particles.

Lamellar structured suspending systems depend on the rheological properties of the suspending medium to immobilize the particles, rather than size of the particles. This requires the suspending medium to exhibit a significant yield point that can counteract the sedimenting or creaming of the suspended particles, but is low enough to enable the medium to flow under externally imposed stresses, such as pouring and stirring, like a normal liquid. The structure reforms sufficiently rapidly to prevent sedimentation, once the agitation caused by the external stress has ceased.

Lamellar structured systems all involve the L[alpha]-phase, in which bilayers of surfactant are arranged with the hydrophobic part of the molecule on the interior and the hydrophilic part on the exterior of the bilayer (or vice versa). The bilayers lie side by side, e.g. in a parallel or concentric configuration, sometimes separated by aqueous layers. La-phases (also known as G-phases) can usually be identified by their characteristic textures under the polarizing microscope and/or by x-ray diffraction, which is often able to detect evidence of lamellar symmetry. Such evidence may comprise first, second and sometimes third order peaks with a d-spacing (2π/Q, where Q is the momentum transfer vector) in a simple integral ratio 1:2:3. Other types of symmetry give non-integral ratios. The d-spacing of the first peak in the series corresponds to the repeat spacing of the bilayer system.

Most surfactants form an Lα-phase either at ambient or at some higher temperature when mixed with water in certain specific proportions. However, these conventional Lα-phases do not function as structured suspending systems. Useful quantities of solid render them unpourable, and smaller amounts tend to sediment.

The main type of structured system used in practice is based on dispersed spherulitic phase. Spherulitic phases comprise well-defined spheroidal bodies, usually referred to in the art as spherulites, in which surfactant bilayers are arranged as concentric shells. The spherulites usually have a diameter in the range 1000 to 15000 angstroms and are dispersed in an aqueous phase in the manner of a classical emulsion. Spherulitic systems are described in more detail in EP 0 151 884.

Most lamellar structured surfactants require the presence of a structurant, as well as surfactant and water in order to form structured systems capable of suspending solids. The term "structurant" is used herein to describe any non-surfactant, capable, when dissolved in water, of interacting with surfactant to form or enhance (e.g. increase the yield point of) a structured system. It is typically a surfactant-desolubiliser, e.g. an electrolyte. However, certain relatively hydrophobic surfactants such as isopropylamine alkyl benzene sulphonate can form spherulites in water in the absence of electrolyte. Such surfactants are capable of suspending solids in the absence of any structurant, as described in EP 0 414 549.

A major problem with lamellar suspending systems, from the point of view of the formulator of personal care products, is that they are formed most readily by surfactant systems that operate as detergents. The higher foaming surfactants (which are most effective in personal care products, such as shampoos) are more soluble in water and are classed as solubilizers.

Attempts to form stable lamellar suspending systems with high foaming surfactant systems have entailed the use of high concentrations of surfactant and high levels of structurant, such as electrolyte or sugar.

In general the use of high surfactant levels, e.g. greater than about 15-20% by weight, is undesirable on grounds both of cost and the potential for producing adverse effects on skin or hair. High electrolyte levels are similarly undesirable, for their potential effects on skin and hair.

Spherulitic surfactant systems induced with high levels of electrolytes are opaque and this limits the visual effects that can be achieved and may be perceived as less attractive than a clear system in some applications. This is because light cannot pass through the dense matrix of spherulites. By contrast many liquids and gases are transparent because light can pass more readily between the large spaces between their atoms. Many crystals are both solid and transparent, this is because the atoms of a crystal are arranged in a precise lattice structure, stacked in regular rows, with regular spacing between them and hence there are many pathways that a light beam can take through a crystal lattice. Solids can also become transparent if their atoms are arranged randomly, glass and sugar candy are good examples.

Transparency can be obtained in certain spherulitic surfactant systems by adding high levels of soluble carbohydrate (e.g. sucrose); but the sugar generally needs to be present in undesirably high concentrations, e.g. over 20% to be effective. Such systems are described in more detail in US2004235702.

There is therefore a need, especially in the personal care field, for a suspending system that contains a blend of high foaming surfactants and is transparent and mobile; but which does not require the presence of electrolyte or sugar as a structurant.

US 6,277,404 B1 is directed to a method of making a product adhere to a surface, wherein the method involves forming microvesicles that encapsulate the product, and then dispersing the microvesicles into an aqueous or lipophilic medium to form a composition that is brought into contact with the surface. WO 2010/054495 A2 relates to non-aqueous colloidal formulations. GB 2464393 A discloses compositions of use in the solubilization of hydrophobic substances and in the solubilization of peptides and proteins for the investigation of their structure and their interactions with other substances. WO 2009/098469 A1 describes structured surfactant systems formed from (i) a major portion of at least one sugar ester or triterpenoid glycoside (saponin) having an HLB greater than 10 and (ii) a minor portion of at least one fatty acid or lecithin. Bendejacq et al. (Cosmet. Toiletries 125 (2010), 22-29) discloses structured surfactant systems for use in shampoo compositions. EP 0 820 755 A2 relates to a composition that can solubilize a hydrocarbon volatilizing agent using a specific coupling agent. WO 2005/053612 A2 describes reverse micellar systems for delivering lipophilic or hydrophobic compounds.

### BRIEF SUMMARY OF THE INVENTION

It has surprisingly been found that transparent structured surfactant suspending systems can be formed by mixing a particular selection of surfactants in particular amounts in the substantial absence of electrolytes or carbohydrates. The transparent structured surfactant systems are particularly useful in personal care compositions to achieve a variety of aesthetically pleasing visual effects. The surfactant systems are mobile, have good suspending power and are phase stable.

In a first aspect, the present technology provides an aqueous structured surfactant system comprising water, and a mixture of surfactants present in an amount of 5% to 40% by weight of the structured surfactant system, wherein the mixture of surfactants comprises at least one surfactant having an HLB value of less than 10, alternatively less than 8, selected from glyceryl caprylate/caprate esters and mixtures thereof; and at least one surfactant having an HLB value of 10 or greater selected from amine oxide, alkyl sulfate, alkyl ether sulfate, alpha-sulpho methyl esters, sodium cholate, alkyl polyglucoside ethoxylated sorbitan esters, sucrose esters, and mixtures thereof, the mixture of surfactants having an overall HLB value in the range of 11 to 13; wherein the structured surfactant system is comprised of multilamellar vesicles with a droplet size of less than 2500 angstroms and a bilayer spacing below 60 angstroms; wherein the structured surfactant system is free of electrolytes and polymeric thickeners; and wherein the structured surfactant system is substantially transparent in the absence of any suspended matter and has suspending properties. In one embodiment of the first aspect, the structured surfactant system further comprises hydroxyl containing compounds having at least two hydroxyl groups. Preferably, the hydroxyl containing compounds comprise glycerol, a polyglycerol or mixtures thereof. In one embodiment of the first aspect, the structured surfactant system further comprises particles of solid, liquid or gas suspended stably within the system. In one embodiment of the first aspect, the ratio of the at least one surfactant having an HLB value of less than 10 to the at least one surfactant having an HLB value of 10 or greater present in the system is from 4:1 to 1:4. In one embodiment of the first aspect, the at least one surfactant having an HLB value of less than 10 is essentially linear and does not possess a bulky hydrophilic headgroup. In one embodiment of the first aspect, the at least one surfactant having an HLB value of less than 10 has a saturated alkyl chain and possess at least one terminal hydroxyl group.

In a second aspect, the present technology provides a composition comprising the structured surfactant system of the first aspect. In one embodiment of the second aspect, the composition is a personal care composition, a pharmaceutical preparation, a laundry composition, a fabric softener composition, an agricultural composition, or a hard surface cleaner.

In a third aspect, the present technology provides a personal care composition comprising: a structured surfactant system comprising water, and a mixture of surfactants in an amount of 5% to 40% by weight of the structured surfactant system wherein the mixture of surfactants comprises at least one surfactant having an HLB value of less than 10 and at least one surfactant having an HLB value of 10 or greater, wherein the at least one surfactant having an HLB value of less than 10 comprises glyceryl caprylate/caprate esters, and the at least one surfactant having an HLB value of 10 or greater comprises amine oxide, alkyl sulfate, alkyl ether sulfate, alkyl polyglucoside, ethoxylated sorbitan esters, sucrose esters, or mixtures thereof; wherein the mixture of surfactants has an overall HLB value in the range of 11 to 13, and produces multilamellar vesicles with a droplet size of less than 2500 angstroms and a bilayer spacing below 60 angstroms; and at least one of solid, liquid or gaseous particles suspended in the structured surfactant system. In one embodiment of the third aspect, the composition further comprises at least one hydroxyl containing compound having at least two hydroxyl groups. Preferably, the hydroxyl containing compounds comprise glycerol, a polyglycerol or mixtures thereof. In one embodiment of the third aspect, the at least one surfactant having an HLB value of less than 10 is essentially linear and does not possess a bulky hydrophilic headgroup. In one embodiment of the third aspect, the at least one surfactant having an HLB value of less than 10 has a saturated alkyl chain and possess at least one terminal hydroxyl group.

### BRIEF DESCRIPTION OF SEVERAL VIEWS OF THE DRAWINGS

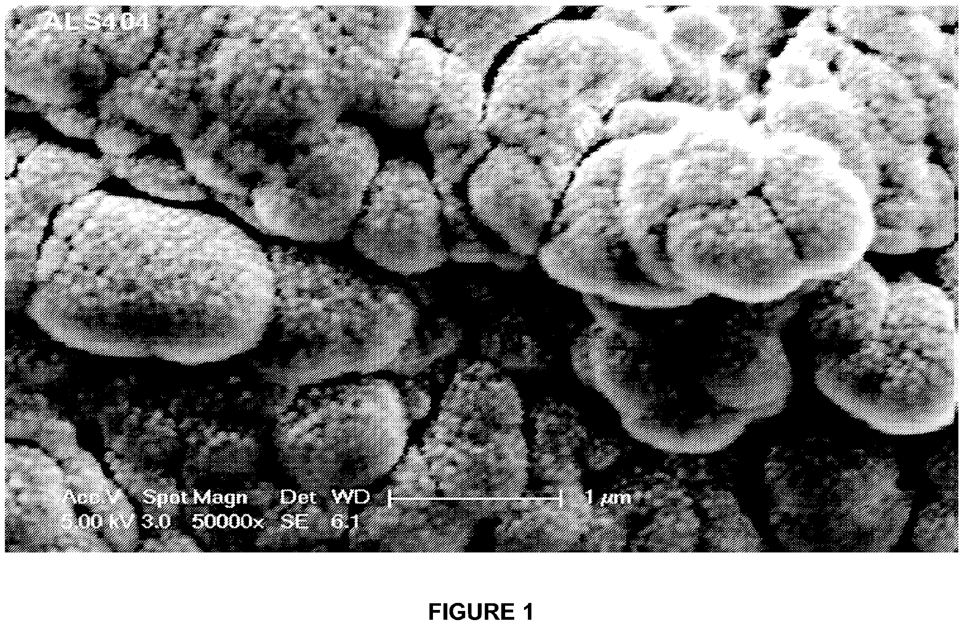

Figure 1 is an electron micrograph illustrating microvesicles of the structured surfactant system.

### DETAILED DESCRIPTION OF THE INVENTION

The present technology relates to a structured suspending system that is based on a lamellar surfactant mesophase. It is transparent, high foaming, can be very low in electrolyte, and can be made without sugar, which makes it ideal for personal care applications. The structured surfactant system comprises water and a mixture of surfactants that form transparent structured liquid compositions in the absence of added electrolytes or carbohydrates. As used herein, the term "transparent" means having the property of transmitting rays of light through its substance so that bodies situated beyond or behind can be distinctly seen. The transparency of the compositions does not preclude the compositions from being colored, for example by the addition of a dye, provided the dye does not detract from the substantial transparency of the composition. Further, the transparency of the compositions does not preclude the compositions from containing various particles to achieve a variety of different effects. For example, mica particles could be suspended in the composition to provide visually pleasing effects, if desired, which would reduce the transparency of the composition. In that case the definition of transparency is applied to the structured liquid composition without the addition of any suspended matter. Transparency can also be measured by methods known in the art. For example, a spectrophotometer can be used to measure the absorption of visible light between 380 and 800 nm. In some embodiments of the present structured surfactant systems, transmission is greater than 50%, more typically greater than 60%.

Analysis of the structured suspending systems (with small angle X-ray, scanning electron microscopy, and rheology) suggests that the majority of the structure units comprise multilamellar microvesicles (i.e. very small spherulites) with around 5-7 concentric shells, alternatively 4-7 concentric shells, and a bi-layer separation of about 45 angstroms. Figure 1 is an electron micrograph showing the microvesicles of the structured surfactant system. The microvesicles are the submicron objects seen on the surface of the large clumps depicted in Figure 1. From the electron micrograph, it is estimated that the microvesicles have a diameter of about 300 to about 1000 angstroms. It is believed that the microvesicles or spherulites are arranged in a precise lattice structure stacked in regular rows, with regular spacing, and this is one possible reason why the compositions have a high degree of transparency and optical clarity. In addition, because the greater proportion of the surfactants that are used contain a terminal hydroxyl group, the spherulites have a refractive index that is close to water. This means that they transmit rather than scatter light, and the refractive index of the continuous medium can be easily matched to give complete transparency by the addition of small amounts of glycerol (for example). This particular structure has hitherto not been identified in the art as a suspending system. Surfactant HLB, headgroup, and shape are all important factors in determining whether a particular blend of surfactants will form this structure. For example, it has been found that the low HLB surfactant must be essentially linear and not possess a bulky hydrophilic headgroup. It may also be important that the low HLB surfactant have a saturated alkyl chain, and possess at least one terminal hydroxyl group.

In so far that it is practicable, it has not been found to be necessary to formulate these systems with any appreciable quantities of electrolyte. It is believed that without electrolyte, the spherulites are dispersed in water and pushed apart into a precise lattice by the electrical charge on the surface of the spherulites (in the case of non-ionic surfactant the charge will be induced by the polarized headgroup). When electrolyte is present it shields the surface charge and allows the spherulites to approach and 'jostle' each other.

The surfactants for use in preparing the structured surfactant system comprise a mixture of at least one surfactant having a hydrophilic-lipophilic balance (HLB) value that is low, i.e. less than 10, and at least one surfactant having a high HLB value, i.e. 10 or greater. Typically, the high HLB value will not exceed 40. Surfactants having a low HLB value are lipophilic and have low solubility in water. Surfactants having a high HLB value are hydrophilic and have a high solubility in water. The combination of surfactants together has an HLB value of 11 to 13, preferably 12.

The surfactants are present in a concentration of 5% to 40% by weight, alternatively 5% to 30% by weight of the structured surfactant system and include any percentage or range there between. Preferred total concentrations of the surfactants range from 7% to 20% alternatively 10% to 15%.

The low HLB surfactant has an HLB value of less than 10, alternatively less than 9, alternatively less than 8, alternatively less than 7, alternatively less than 6, alternatively less than 5. Surfactants having a low HLB value are glyceryl fatty acid esters. Such glyceryl fatty acid esters are made by esterifying glycerin with medium to long chain fatty acids having from 6 to 18 carbon atoms. The resulting product comprises a mixture of mono-, di-, and/or triglycerides of fatty acids, the relative proportions depending on the process used and the ratio of reactant employed. Preferred glyceryl fatty acid esters are mixtures of mono- and di-glycerides of caprylic and capric acids. A commercially available example of glyceryl caprylate/caprate can be obtained from Stepan Company, Northfield, Illinois, under the tradename Stepan-Mild® GCC. The HLB value of Stepan-Mild® GCC is in the range of about 5 to about 6. Mixtures of the low HLB surfactants can be utilized. For example, fatty alcohols at levels of about 1% by weight in combination with glycerol esters provide a foam boosting/enhancing effect.

The high HLB surfactant has an HLB value of 10 or greater and can be anionic, cationic, or nonionic. Suitable anionic surfactants are alpha-sulfo methyl esters, alkyl sulfates, alkyl ether sulfates, and sodium cholate.

Suitable nonionic surfactants are alkyl polyglucosides, ethoxylated sorbitan esters, and sucrose esters.

The high HLB surfactant can also be amine oxide preferably having a carbon chain length in the range of 10-18 carbons. Mixtures of high HLB surfactants may also be employed. Preferred high HLB surfactants are those that contain no or minimal levels of electrolyte. Alkyl sulfates and alkyl ether sulfates are particularly suitable high HLB surfactants.

The low and high HLB surfactants are mixed together in relative proportions in order to obtain a resulting HLB of the mixture in the range of 11 to 13, preferably 12. In general, the ratio of the low HLB surfactant to high HLB surfactant in the surfactant system can range from 4:1 to 1:4, depending upon the particular surfactants selected.

The structured surfactant system can further include a component to adjust the refractive index to improve the optical clarity of the system. Without being bound by theory, it is believed that when appropriate ratios of the low HLB surfactant and the high HLB surfactant are mixed together, the mixture of surfactants forms liquid crystal dispersions composed of spherulites arranged in a precise lattice structure in the dispersing medium, for example water. Adding a component to the structured surfactant system to adjust the refractive index of the continuous, dispersing medium so that it is closer to that of the spherulites, results in improved optical clarity of the structured surfactant system.

Suitable materials for adjusting the refractive index include polyhydroxy compounds having at least two hydroxyl groups, such as, for example, ethylene glycol, propylene glycol, butylene glycol, glycereth-7, glycerol, polyglycerol, trimethylolpropane, pentaerythritol, and sorbitol. Glycerol is a preferred material and mixtures of glycerol and polyglycerol (e.g. triglycerol) are also preferred. It is believed that as well as altering the refractive index, polyglycerols also improve clarity by promoting the formation of small spherulites. Mixtures of glycerol and polyglycerols show synergism since they are around twice as effective at clarifying compositions as either glycerol or polyglycerol alone. The amount of material added will depend upon how much adjustment is needed. In general, the amount of hydroxyl-containing material added will range from about 1% to about 15% by weight, alternatively, about 1% to about 10% by weight of the structured surfactant system. More usually the amount will be between about 2% to about 6% by weight of the structured surfactant system. Blends of 50% glycerol plus 50% polyglycerol (e.g. triglycerol) have been found to be particularly effective.

It has further been discovered that clarity may be enhanced by incorporating a proportion of polyoxyethylene groups. These have been found to function when they are present as simple polyoxyethylene chains, i.e. polyethylene glycols; and also when they comprise the hydrophilic component of a surfactant, e.g. ethoxylated sorbitan ester. For example, in some embodiments, amounts of 0.5% PEG (as PEG 400) and 1% of POE (20) sorbitan monolaurate have been found to be effective.

The structured surfactant system of the present technology has good yield value. By "yield value" is meant that the structured surfactant system has the ability to support particulate (gas, solid, liquid) matter. The yield value enables the structured surfactant system to suspend solid, liquid or gas particles throughout a liquid composition.

The composition may contain suspended solid, liquid or gaseous particles. For instance the composition may contain suspended oil droplets. The oil is preferably a mineral oil (e.g. a low molecular weight petroleum oil) or a fatty glyceride or other ester such as lauryl acetate, a terpene oil such as limonene or a silicone oil. Mixtures of oils may be used. Particularly preferred are vegetable oils such as coconut, evening primrose, groundnut, meadow foam, apricot kernel, peach kernel, avocado, jojoba and olive oil. Oil soluble cosmetic or topical pharmaceutical ingredients may be dissolved in the oil including antiseptics, styptics, antidandruff agents such as zinc omadine (zinc pyrithione) and selenium disulphide, proteins, emollients such as lanolin, isopropyl myristate, glyceryl isostearate or propylene glycol distearate, dyes, perfumes and waxes. Water insoluble particulate solids may be suspended including exfoliants such as talc, clays, polymer beads, sawdust, silica, seeds, ground nutshells and dicalcium phosphate, pearlizers such as mica or glycerol or ethylene glycol di-stearate, glitter additives and sunscreens such as titanium dioxide and zinc oxide. Porous particles (so called micro-sponges) containing absorbed active ingredients or gelatin or other microcapsules may also be suspended. Other active ingredients which may be suspended include insect repellents and topical pharmaceutical preparations, e.g. preparations for treatment of acne, fungicides for athlete's foot or ringworm or antiseptics or antihistamines. Pigments, such as the iron oxides, may also be added. The particle size of the suspended material can vary widely, for example from about 5 microns to several millimeters in size.

The suspended particles can be added to personal care compositions for purely aesthetic reasons to achieve visually pleasing effects. Alternatively, the structured surfactant systems can be used in other applications where water insoluble or sparingly soluble components are utilized, such as in laundry and fabric softener compositions, agricultural compositions containing pesticides and insecticides, pharmaceutical suspensions, and hard surface cleaners containing abrasives, for example calcium carbonate.

The structured surfactant systems can be prepared by mixing the components at room temperature, then storing overnight at a temperature of about 55°C, alternatively about 45°C. The components are then remixed and cooled to room temperature. When mixing the compositions it is important to sequence the order of mixing to avoid the formation of liquid/liquid emulsions. These will tend to form if the oil soluble surfactants are added to a stirred solution of the high HLB surfactants. Liquid/liquid emulsions are metastable suspensions of liquid droplets, whereas the structured surfactant systems described herein are typically stable dispersions of liquid crystals. To avoid forming liquid/liquid emulsions it is preferred that all the surfactants are blended together to give a high active concentrate before they are added to the water. The high active concentrates have been found to be pourable and to disperse readily with low shear mixing at room temperature to form the structured surfactant. The resulting structured surfactant systems are transparent, and flowable, and achieve good suspending ability without the addition of electrolytes, carbohydrates or polymeric thickeners. They can be used alone as a liquid cleansing composition, for example as a body wash, hand wash, shampoo or the like. Alternatively, other optional ingredients may be added to make the structured surfactant systems suitable for a variety of different uses, such as a water-based scrub, a textile wash/softener and the like.

Optional ingredients include fatty acid soaps, builders, and additional surfactants to aid in cleaning ability. Emollients (including, without limitation, vegetable oils, mineral oils, silicone oils, petrolatum, polyglycerol methyl esters and esters), skin conditioning agents, vitamins and herbal extracts can be added to further improve conditioning performance. Fragrances and dyes may also be added to further enhance the appearance and smell of the finished product. Suitable preservatives, such as benzyl alcohol, methyl paraben, propyl paraben, methylchloroisothiazolinone/methyl-isothiazolinone, phenoxyethanol, imidazolidinyl urea and DMDM hydantoin, may be utilized.

If necessary, additional thickeners may be added to achieve a desired viscosity for a particular composition. Such thickening agents may include, for example, esterquats, amidoquats, steramidopropyl dimethyl amine quat and polymeric thickeners such as cellulosic polymers, acrylic polymers, and hydroxyl propyl guar gum.

When formulated for personal care, the compositions containing the structured surfactant system typically have a pH of between about 4.0 to about 8.5, alternatively between about 5.0 to about 7.0. Techniques for controlling pH at recommended usage levels include the use of buffers, alkali and acids and are known to those skilled in the art. Optional pH adjusting agents can include, but are not limited to, citric acid, succinic acid, phosphoric acid, sodium hydroxide, sodium carbonate, and the like.

### Examples

The following examples describe some of the preferred embodiments of the present technology without limiting the technology thereto. Other embodiments include, but are not limited to, those described in the above written description, including additional or alternative components, alternative concentrations, and additional or alternative properties and uses. In the following examples, all proportions are based on weight percentages of active ingredients based on the total weight of the composition, unless stated to the contrary.

**Table A: Composition Trade Names & Abbreviations**

| | |
|---|---|
| ALPHA-STEP® PC-48 | sodium methyl 2-sulfolaurate and disodium 2-sulfolaurate |
| STEOL®CS-270-E | sodium salt of C₁₂-C₁₄ alkyl ethoxy sulfate with 2 moles ethylene oxide per mole of alcohol |
| STEPANOL® ALS 25 | Ammonium lauryl sulphate |
| AMMONYX® LO | Lauramine oxide |
| AMMONYX® LMDO | Lauramidopropylamine oxide |
| STEPAN-MILD® GCC | Glyceryl caprylate/caprate |
| PLANTACARE® 818UP | C₈-C₁₆ fatty alcohol glucoside |
| NINOL® 40C0-E | Cocamide diethanolamine |
| NINOL®COMF-N | Cocamide monoethanolamine |

ALPHA-STEP® PC-48, STEOL® CS-270-E, AMMONYX® LO, AMMONYX® LMDO, NINOL®COMF-N, NINOL® 40C0-E, STEPANOL® ALS 25 and STEPAN-MILD® GCC are commercially available from Stepan Co., Northfield, IL. PLANTACARE® 818UP is commercially available from BASF/Cognis.

### Example 1

A series of formulations were prepared by mixing Ammonyx® LO amine oxide, and Stepan-Mild® GCC glyceryl caprylate/caprate in ratios of amine oxide to glyceryl caprylate/caprate ranging from 1:3 to 2:1. Total surfactant for each formulation was 15% by weight. Table B shows the ratio of each surfactant and the visual results for each formulation.

**Table B: Ammonyx® LO Plus Stepan Mild® GCC w/w Blends in Water 15% Total Surfactant**

| Amine Oxide : GCC Surfactant Ratio | Result |
|---|---|
| | Opaque liquid - about 33% v/v |
| 1:3 | Clear liquid - about 7% v/v |
| | Clear liquid - about 60% v/v |
| 1:2 | Cloudy liquid - about 10% v/v |
| | Clear liquid - about 90% v/v |
| 1:1.5 | Structured liquid with high degree of clarity that suspends bubbles - 100% |
| 1.25:1 | Structured liquid with high degree of clarity that suspends bubbles - 100% |
| 1.5:1 | Turbid liquid - about 80% v/v |
| | Clear liquid - about 20% v/v |
| 2:1 | Turbid liquid - about 10% v/v |
| | Clear liquid - about 90% v/v |

Structured liquids were obtained over the ratio of 1:1.5 to 1.25:1 amine oxide to glyceryl caprylate/caprate, with clarity increasing with increased amount of amine oxide. The highest degree of clarity was obtained at 1.25:1 w/w amine oxide : glyceryl caprylate/caprate

### Example 2

Glycerol was added to blends of 1.25:1 w/w amine oxide : glyceryl caprylate/caprate to determine whether added glycerol could improve the optical clarity of the blends. Table C shows the formulations prepared with added glycerol.

**Table C: Formulations with Added Glycerol**

| Component % w/w | Sample 1(a) | Sample 1(b) | Sample 1(c) |
|---|---|---|---|
| Amine oxide (Ammonyx® LO) | 8.33 | 8.33 | 8.33 |
| Stepan-Mild® GCC | 6.66 | 6.66 | 6.66 |
| Glycerol | 1.00 | 5.00 | 10.00 |
| Water | Bal | Bal | Bal |

The formulations were prepared by mixing all the components by hand at room temperature and storing overnight at 55°C, re-stirring, and then cooling to room temperature. All the formulations were structured liquids that suspended air. There was a noticeable difference in clarity between the samples. The sample with 5% glycerol was the clearest, with a very high degree of clarity, followed by the sample with 10% glycerol, then the sample with 1% glycerol. These results show that adjusting the refractive index of the continuous phase can improve the clarity of liquid crystal dispersions.

### Example 3: Glyceryl Caprylate/Caprate with Other Surfactants

Formulations were prepared by mixing glyceryl caprylate/caprate with different hydrophilic surfactants and dispersing in water. Glycerol was incorporated as necessary, to adjust the refractive index. The different formulations are shown in Table D.

**Table D**

| | 2 | 3 | 4 | 5 | 6 | 7 |
|---|---|---|---|---|---|---|
| Component % w/w | | | | | | |
| STEPAN-MILD® GCC | 11.25 | 11.25 | 10.0 | 6.66 | 9.72 | 7.14 |
| Lauramine oxide (ex Ammonyx® LO) | | | | 8.33 | | |
| Sodium methyl 2-sulfolaurate and disodium 2-sulfolaurate(ex ALPHA-STEP® PC-48) | 3.75 | - | - | | | |
| Sodium laureth (2EO) sulphate (ex STEOL® CS-270-E) | - | - | 2.5 | | 3.95 | |
| Ammonium Lauryl Sulfate (ex STEPANOL® ALS 25) | - | 3.75 | - | | | |
| Alkyl polyglucoside (ex Plantacare® 818UP) | | | | | | 7.13 |
| Glycerol | 5.0 | - | 5.0 | 5.0 | 5.0 | 4.76 |
| Water | Balance | Balance | Balance | Balance | Balance | Balance |

Each of the formulations was prepared by mixing the components together by hand at room temperature, storing overnight at 55 °C, re-stirring, and then cooling to room temperature. Sample 2, comprising a ratio of about 3:1 w/w GCC : Alpha-Step® PC 48 and total surfactant of about 15% by weight, provides a strong structured surfactant suspending system that is only slightly turbid. Sample 3, comprising a ratio of about 3:1 GCC : ALS and total surfactant of about 15% by weight also provides a strong structured surfactant suspending system that is slightly turbid. Sample 4, comprising a ratio of about 4:1 GCC : SLES and total surfactant of about 12.5% by weight is effectively transparent.

### Example 4: Concentrated Blend

The following concentrated blend was prepared based on the Sample 1(b) amine oxide/GCC formulation of Example 2.

| |
|---|
| 25.00g Glycerol |
| 33.33g Stepan-Mild®GCC |
| 138.88g Ammonyx®LO |

The resulting blend is a turbid "thin lamellar" phase - very mobile at 50% w/w solids, 38% total surfactant. When 40g of this blend is gently stirred into 60g of water at room temperature a transparent structured system is readily formed.

### Example 5

Formulations were prepared utilizing the structured surfactant suspending systems to suspend beads in the compositions. The formulations are shown in Table E. The formulations were found to be phase stable and non-sedimenting after storing for 3 months at room temperature and also at 40°C and 3°C.

**Table E**

| Component %w/w | Formula 1 | Formula 2 |
|---|---|---|
| Sodium laureth sulfate (2 EO) ex STEOL® CS-270-E (Stepan) | 2.5 | |
| STEPAN-MILD® GCC (Stepan) | 10.0 | 6.66 |
| Amine Oxide ex.Ammonyx®LMDO (Stepan) | | 6.66 |
| Ninol® 40C0-E - coconut diethanolamide (Stepan) | | 6.66 |
| Glycerol | 5.0 | |
| Perfume | | 0.5 |
| Blue Unispheres NT 2103 (Induchem USA) | 0.5 | 0.5 |
| Preservative Kathon CG (Dow) | 0.02 | 0.02 |
| Viscosity (mPa·s (cps))@ 23 Deg. Centigrade (Brookfield RVT, spindle 5, speed 100) | 2020 (2020) | 2086 (2086) |
| Appearance | Transparent base with suspended beads | Transparent base with suspended beads |

Formula 1 was prepared by firstly dissolving the sodium laureth sulfate (2 EO) in water; heating to 60°C with gentle stirring; adding glycerol, then Kathon, then beads, and finally the GCC. Gentle stirring was maintained throughout; agitation was stopped as soon as the sample thickened. Formula 2 was prepared by mixing amine oxide with water and Kathon and heating to 60°C. The remaining ingredients were mixed together at room temperature before adding the hot amine oxide solution to this mix with gentle agitation and stirring until the sample thickened. Before bottling, both Formula 1 and Formula 2 were thermostated at 60°C for 16 hrs.

Formula 1 (minus the beads) was found to have a viscosity of ca. 2000 mPa·s (2000 cps) at 22°C when measured on a Brookfield RVT viscometer at 21 reciprocal seconds. Viscosity (at the same temperature) was then measured over the range 0.1 to 100 reciprocal seconds on a Mettler RM260 rheometer. The sample was found to be shear thinning and showed a quick recovery back to the starting viscosity on cessation of shear.

### Example 6

A handwash formulation was prepared utilizing the structured surfactant suspending system and mica and glitter was suspended in it. The composition is shown in Table F. It was found to be phase stable and non-sedimenting after storing for 3 Months at room temperature and also at 40°C and 3°C. It was found to foam well in use and leave the hands feeling soft and moisturized.

**Table F**

| Component %w/w | Composition A with mica | Composition B with glitter |
|---|---|---|
| STEPAN-MILD® GCC (Stepan) | 5.0 | 5.0 |
| Amine Oxide ex.Ammonyx LMDO® (Stepan) | 5.0 | 5.0 |
| Ninol 40C0-E® - coconut diethanolamide (Stepan) | 4.5 | 4.5 |
| Tetraglycerol monooleate | 0.5 | 0.5 |
| Perfume | 0.5 | 0.5 |
| Mica "Bright Gold" Colorona® (Merck) | 0.05 | |
| Glitter "Silver Flash" 15-150 microns (Sumicos) | | 0.05 |
| Preservative Kathon GC® (Dow) | 0.02 | 0.02 |
| Viscosity (mPa·s (cps))@ 23 Deg. Centigrade (Brookfield RVT, spindle 5, speed 100) | 1084 (1084) | 1032 (1032) |
| Appearance | Visually pleasing swirls of gold speckles | Visually pleasing swirls of glitter |

The samples were prepared by mixing amine oxide with water and Kathon and heating to 60°C. The remaining ingredients were mixed together at room temperature before adding the hot amine oxide solution to this mix with gentle agitation and stirring until the sample thickened. Before bottling, the samples were thermostated at 60°C for 16 hrs.

### Example 7

Bodywash formulations containing suspended oil droplets were prepared. The compositions are shown in table G. They were found to be phase stable with no separation after storing for 3 months at room temperature and also at 40°C and 3°C. They foamed surprisingly well in use and delivered oil onto the skin to provide a unique "two in one" cleansing/moisturizing experience.

**Table G**

| Component (%w/w) | Formula 1 | Formula 2 |
|---|---|---|
| STEPAN-MILD® GCC (Stepan) | 12.85 | 14.33 |
| Glycerol | | 2.0 |
| Paraffinum Liquidum | 20.0 | 2.5 |
| Refined Shea Butter | | 0.2 |
| Perfume Hypo 301 (Givaudan) | | 0.2 |
| ALPHA-STEP® PC-48 (alpha sulfo methyl ester blend ex Stepan) | | 1.84 |
| Ammonium lauryl sulfate ex STEPANOL® ALS 25 (Stepan) | 4.81 | 2.75 |
| Citric acid | | 0.044 |
| Trisodium citrate | | 0.224 |
| Preservative Kathon GC (Dow) | 0.02 | 0.02 |
| Viscosity (mPa·s (cps))@ 23 Deg. Centigrade (Brookfield RVT, spindle 5, speed 100) | | 1936 (1936) |
| Comments | Demonstration bodywash chassis containing a very high level of oil. Opaque mousse. | Optimized bodywash. Opaque pourable liquid. |

Formula 2 was prepared by mixing together the GCC, paraffinum liquidum, shea butter, and perfume and warming gently (30°C) until a clear solution was obtained. This mixture was then poured into a stirred solution of the remaining components at room temperature. The sample was stirred efficiently (without entraining air) until it was smooth and homogeneous.

Formula 2 was examined by small angle X-ray, count time 600 seconds, room temperature. The x-ray results show weak scattering with a hump around 45 angstroms; this is consistent with microvesicles that contain only a few concentric shells.

Formula 2 was analyzed by freeze fracture electron microscopy; the results are consistent with a droplet size less than 2500 angstroms in diameter. Calculations indicate that spherulites with 5-7 shells and a bilayer separation of 45 angstrom will have a size of the order of ca. 500 angstroms. It is difficult to fully resolve structure units around 500 angstroms using this technique.

### Example 8

A glyceryl caprylate/caprate and ammonium lauryl sulphate structured surfactant system was used to prepare two water-based sugar scrub compositions. Typically sugar and salt scrubs are predominantly oil based, and a water-based scrub offers certain advantages. The scrubs are listed in Table H. They were found to be phase stable with no separation after storing for 1 month at room temperature and also at 45°C and 0°C.

**Table H**

| Component (%w/w) | Formula A | Formula B |
|---|---|---|
| STEPAN-MILD® GCC (Stepan) | 6.63 | 4.86 |
| Ammonium lauryl sulfate ex STEPANOL® ALS 25 (Stepan) | 2.21 | 1.62 |
| Sucrose (granulated white table sugar) | 70.3 | 70.0 |
| Soybean oil | | 4.53 |
| Shea butter | | 0.4 |
| Perfume | 0.4 | 0.4 |
| Citric acid | 0.014 | 0.014 |
| Trisodium citrate | 0.083 | 0.083 |
| water | balance | balance |
| pH | 5.7 | 5.7 |

The scrubs were prepared by charging all the components to the water then stirring gently at room temperature until a smooth homogeneous consistency was obtained.

### Example 9

An antidandruff shampoo was formulated (Table J) containing 1% zinc omadine. It was found to be phase stable with no separation after storing for 3 months at room temperature and also at 45°C and 0°C.

**Table J**

| Component (%w/w) | |
|---|---|
| STEPAN-MILD® GCC (Stepan) | 9.50 |
| Ammonium lauryl sulfate ex STEPANOL® ALS 25 (Stepan) | 3.27 |
| Ninol® coconut monoethanolamide (Stepan) | 0.55 |
| Sodium laureth sulfate (2 EO) ex STEOL® CS-270-E (Stepan) | 0.56 |
| Zinc omadine slurry - 48% aqueous dispersion (Arch) | 2.4 |
| Perfume | 0.5 |
| Preservative Kathon CG (Dow) | 0.02 |
| water | balance |
| Product aspect | Dense white opaque liquid |
| Viscosity (mPa·s (cps))@ 23 Deg. Centigrade (Brookfield RVT, spindle 4, speed 100) | 1480 (1480) |

The composition was prepared at room temperature with a low shear paddle stirrer by dissolving the Ninol® into the GCC, glyceryl caprylate/caprate then, adding to this the ammonium lauryl sulfate, the sodium laureth sulfate, water, zinc omadine, Kathon, and finally perfume; maintaining gentle shear without entraining an excessive amount of air.

### Example 10

Various compounds were found to have a good clarifying effect on the basic glyceryl caprylate/caprate and ammonium lauryl sulphate structured surfactant system. Without clarifying agents the composition is slightly turbid. Microscopic examination (polarizing microscope) indicates that the turbidity is due to large extremely faint spherulites. It is believed that the clarifying agents listed below operate by either refractive index matching, or by reducing spherulite size, or (certainly in the case of polyglycerols) by a combination of both mechanisms.

**Table K**

| Component (% w/w) | A | B | C | D | E | F | G |
|---|---|---|---|---|---|---|---|
| STEPAN-MILD® GCC (Stepan) | 9 | 9 | 9 | 9.6 | 10.6 | 9.6 | 9.6 |
| Ammonium lauryl sulfate | 3 | 3 | 3 | 3.85 | 3.85 | 3.85 | 3.85 |
| ex STEPANOL® ALS 25 (Stepan) | | | | | | | |
| Glycerol | 10 | | 2 | 6 | 6 | 6 | 6 |
| Triglycerol | | 6 | 2 | | | | |
| Cetyl stearyl alcohol | | | | 0.45 | 0.45 | 0.45 | 0.45 |
| POE (20) sorbitan monooleate | | | | 2.0 | | | |
| POE (20) sorbitan monolaurate | | | | | 1.0 | | |
| C12/14 alcohol + 50 moles EO | | | | | | 0.5 | |
| PEG400 | | | | | | | 0.5 |
| Order of clarity by visual comparison | 4 | 1 | 1 | 3 | 2 | 3 | 4 |
| (1 = clearest) | | | | | | | |
| Viscosity (mPa·s (cps))@ 23 Deg. Centigrade | 1480 (1480) | 1494 (1494) | 1476 (1476) | 3904 (3904) | 2840 (2840) | 2084 (2084) | 1936 (1936) |
| (Brookfield RVT, spindle 5, speed 100) | | | | | | | |

Samples are prepared by blending surfactants and additives together at room temperature to give a fluid lamellar 'paste', then adding water and stirring gently until the structured surfactant system forms. Samples are then thermostated at 45°C for 16 hours. All the samples have a very high degree of clarity but Formulations B and C in Table K are markedly superior - they are almost crystal clear. A combination of glycerol and triglycerol gives the best performance for at least the additives. Samples of narrow cut polyglycerides (-3, -4, and -6 ex Spiga Nord S.P.A.) were examined in the ALS/GCC/Glycerol system. No significant differences were found between them in clarifying effect.

### Example 11

Glyceryl caprylate/caprate (Stepan-Mild® GCC) is an approved excipient for oral pharmaceutical formulations. By combining it with high HLB pharmaceutical excipients, several suspension systems useful for formulating insoluble drug particles can be constructed. The ratios of high HLB excipient to Stepan-Mild® GCC required to construct the suspending systems for a number of excipients were determined experimentally and are listed in Table L.

**Table L**

| Sample | High HLB excipient | Ratio of excipient to Stepan-Mild® GCC w/w | % Actives |
|---|---|---|---|
| A | Sucrose stearate (HLB 15) | 1:1 | 15.0 |
| B | POE(20) sorbitan monolaurate | 1:1 | 15.0 |
| C | POE(20) sorbitan monooleate | 1:1.25 | 15.0 |
| D | Sodium cholate | 1:9.5 | 15.0 |

Samples are prepared by low-shear mixing all components together at room temperature; thermostating for 16 hours at 45°C then remixing prior to cooling to room temperature. All of the above examples are slightly turbid liquids with viscosities around 2000 mPa·s (2000 cps) @ 21 reciprocal seconds. They are birefringent when the bulk sample is viewed through polarizing filters and will suspend particles of solid, liquid or gas.

### Example 12 (reference example)

Lauramine oxide was combined with 2.5 and 3.0 mole linear ethoxylated alcohols to produce systems with good potential for suspending agrochemical actives. The ethoxylated alcohols chosen were C11 plus 3EO and C9-11 plus 2.5EO. Table M lists the ratio of lauramine oxide to alcohol ethoxylate that gives a suspending system.

**Table M**

| Ethoxylated Alcohol | Ratio of lauramine oxide to ethoxylated alcohol (w/w) | % Actives |
|---|---|---|
| C9-11 oxo alcohol (75% linear) plus 2.5EO (Hydroxyl value 203 mg KOH/g) | 1:1.175 | 15.0 |
| C11 oxo alcohol (75% linear) plus 3EO (Hydroxyl value 185 mg KOH/g) | 1:2.750 | 15.0 |

Samples are prepared by low-shear mixing all components together at room temperature. They are slightly turbid liquids with viscosities around 2000 mPa·s (2000 cps) @ 21 reciprocal seconds and will suspend particles of solid, liquid or gas.

### Example 13 (reference example)

A suspending system was prepared for use with an agricultural active to prepare an agricultural composition. A chlorothalonil dispersion was prepared by blending the surfactants shown in Table N together before dispersing them in water (at room temperature) and then gently stirring in the chlorothalonil active until a smooth homogeneous sample was obtained. The composition having the formulation shown in Table N was opaque and pourable. It was stable for 3 months at room temperature and showed no signs of sedimentation and/or phase separation.

**Table N**

| Component | (%w/w) |
|---|---|
| C12/C14 fatty alcohol plus 2EO | 3.4 |
| Amine Oxide ex. Ammonyx LO® (Stepan) | 2.5 |
| Chlorothalonil (unmilled tech grade 98.1% ex. Sorn Dynamics) | 40.0 |

| Demineralised water | balance |
|---|---|
| Product aspect | Dense off white opaque liquid |
| Viscosity (mPa·s (cps))@ 23 Deg. Centigrade (Brookfield RVT, spindle 4, speed 100) | 2000 (2000) |

## Claims

1. An aqueous structured surfactant system comprising:
water, and a mixture of surfactants present in an amount of 5% to 40% by weight of the structured surfactant system, wherein the mixture of surfactants comprises:
at least one surfactant having an HLB value of less than 10 selected from glyceryl caprylate/caprate esters and mixtures thereof; and
at least one surfactant having an HLB value of 10 or greater selected from amine oxide, alkyl sulfate, alkyl ether sulfate, alpha-sulpho methyl esters, sodium cholate, alkyl polyglucoside, ethoxylated sorbitan esters, sucrose esters, and mixtures thereof, the mixture of surfactants having an overall HLB value in the range of 11 to 13;
wherein the structured surfactant system is comprised of multilamellar vesicles with a droplet size of less than 2500 angstroms and a bilayer spacing below 60 angstroms;
wherein the structured surfactant system is free of electrolytes and polymeric thickeners; and
wherein the structured surfactant system is substantially transparent in the absence of any suspended matter and has suspending properties.

2. The aqueous structured surfactant system of claim 1, wherein the structured surfactant system further comprises hydroxyl containing compounds having at least two hydroxyl groups.

3. The aqueous structured surfactant system of claim 2, wherein the hydroxyl containing compounds comprise glycerol, a polyglycerol or mixtures thereof.

4. The aqueous structured surfactant system of claims 1-3, wherein the structured surfactant system further comprises particles of solid, liquid or gas suspended stably within the system.

5. The aqueous structured surfactant system of claims 1-4, wherein the ratio of the at least one surfactant having an HLB value of less than 10 to the at least one surfactant having an HLB value of 10 or greater present in the system is from 4:1 to 1:4.

6. The aqueous structured surfactant system of claims 1-5, wherein the at least one surfactant having an HLB value of less than 10 is essentially linear and does not possess a bulky hydrophilic headgroup.

7. The aqueous structured surfactant system of claims 1-6, wherein the at least one surfactant having an HLB value of less than 10 has a saturated alkyl chain and possess at least one terminal hydroxyl group.

8. A composition comprising the structured surfactant system of any of claims 1-7.

9. The composition of claim 8, wherein the composition is a personal care composition, a pharmaceutical preparation, a laundry composition, a fabric softener composition, an agricultural composition, or a hard surface cleaner.

10. A personal care composition comprising:
a structured surfactant system comprising water, and a mixture of surfactants in an amount of 5% to 40% by weight of the structured surfactant system, wherein the mixture of surfactants comprises at least one surfactant having an HLB value of less than 10 and at least one surfactant having an HLB value of 10 or greater, wherein the at least one surfactant having an HLB value of less than 10 comprises glyceryl caprylate/caprate esters, and the at least one surfactant having an HLB value of 10 or greater comprises amine oxide, alkyl sulfate, alkyl ether sulfate, alkyl polyglucoside, ethoxylated sorbitan esters, sucrose esters, or mixtures thereof; wherein the mixture of surfactants has an overall HLB value in the range of 11 to 13, and produces multilamellar vesicles with a droplet size of less than 2500 angstroms and a bilayer spacing below 60 angstroms; and
at least one of solid, liquid or gaseous particles suspended in the structured surfactant system.

11. The personal care composition of claim 10, wherein the composition further comprises at least one hydroxyl containing compound having at least two hydroxyl groups.

12. The personal care composition of claim 11, wherein the hydroxyl containing compound comprises glycerol, a polyglycerol or mixtures thereof.

13. The personal care composition of claims 10-12, wherein the at least one surfactant having an HLB value of less than 10 is essentially linear and does not possess a bulky hydrophilic headgroup.

14. The personal care composition of claims 10-13, wherein the at least one surfactant having an HLB value of less than 10 has a saturated alkyl chain and possess at least one terminal hydroxyl group.

## Patentansprüche

1. Wässriges, strukturiertes Tensidsystem, umfassend:
Wasser und ein Gemisch von Tensiden, das in einer Menge von 5% bis 40% nach Gewicht des strukturierten Tensidsystems vorhanden ist, wobei das Gemisch von Tensiden:
mindestens ein Tensid, das einen HLB-Wert von weniger als 10 aufweist und aus Glycerylcaprylat-/-capratestern und Gemischen davon ausgewählt ist; und
mindestens ein Tensid, das einen HLB-Wert von 10 oder mehr aufweist und aus Aminoxid, Alkylsulfat, Alkylethersulfat, alpha-Sulphomethylestern, Natriumcholat, Alkylpolyglucosid, ethoxylierten Sorbitanestern, Sucroseestern und Gemischen davon ausgewählt ist, wobei das Gemisch von Tensiden einen Gesamt-HLB-Wert im Bereich von 11 bis 13 aufweist;
umfasst,
wobei das strukturierte Tensidsystem aus multilamellaren Vesikeln mit einer Tropfengröße von weniger als 2500 Angström und einem Doppelschicht-Abstand von unter 60 Angström aufgebaut ist;
wobei das strukturierte Tensidsystem frei von Elektrolyten und polymeren Verdickungsmitteln ist; und
wobei das strukturierte Tensidsystem transparent in der Abwesenheit eines jegliches suspendierten Materials ist und Suspendiereigenschaften aufweist.

2. Wässriges, strukturiertes Tensidsystem nach Anspruch 1, wobei das strukturierte Tensidsystem ferner hydroxylgruppenhaltige Verbindungen mit mindestens zwei Hydroxylgruppen umfasst.

3. Wässriges, strukturiertes Tensidsystem nach Anspruch 2, wobei die hydroxylgruppenhaltigen Verbindungen Glycerin, ein Polyglycerin oder Gemische davon umfassen.

4. Wässriges, strukturiertes Tensidsystem nach den Ansprüchen 1-3, wobei das strukturierte Tensidsystem ferner Partikel eines Feststoffes, einer Flüssigkeit oder eines Gases, stabil in dem System dispergiert, umfasst.

5. Wässriges, strukturiertes Tensidsystem nach den Ansprüchen 1-4, wobei das Verhältnis des mindestens einen in dem System vorhandenen Tensids mit einem HLB-Wert von weniger als 10 zu dem mindestens einen in dem System vorhandenen Tensid mit einem HLB-Wert von 10 oder mehr 4:1 bis 1:4 beträgt.

6. Wässriges, strukturiertes Tensidsystem nach den Ansprüchen 1-5, wobei das mindestens eine Tensid mit einem HLB-Wert von weniger als 10 linear ist und keine voluminöse hydrophile Kopfgruppe aufweist.

7. Wässriges, strukturiertes Tensidsystem nach den Ansprüchen 1-6, wobei das mindestens eine Tensid mit einem HLB-Wert von weniger als 10 eine gesättigte Alkylkette aufweist und über mindestens eine terminale Hydroxylgruppe verfügt.

8. Zusammensetzung, die das strukturierte Tensidsystem nach einem jeglichen der Ansprüche 1-7 umfasst.

9. Zusammensetzung nach Anspruch 8, wobei die Zusammensetzung eine Körperpflegezusammensetzung, eine pharmazeutische Zubereitung, eine Waschzusammensetzung, eine Weichspülerzusammensetzung, einer Agrarzusammensetzung oder ein Reiniger für harte Oberflächen ist.

10. Körperpflegezusammensetzung, umfassend:
ein strukturiertes Tensidsystem, das Wasser und ein Gemisch von Tensiden in einer Menge von 5% bis 40% nach Gewicht des strukturierten Tensidsystems umfasst, wobei das Gemisch von Tensiden mindestens ein Tensid mit einem HLB-Wert von weniger als 10 und mindestens ein Tensid mit einem HLB-Wert von 10 oder mehr aufweist, wobei das mindestens eine Tensid mit einem HLB-Wert von weniger als 10 Glycerylcaprylat-/-capratester umfasst und das mindestens eine Tensid mit einem HLB-Wert von 10 oder mehr Aminoxid, Alkylsulfat, Alkylethersulfat, Alkylpolyglucosid, ethoxylierte Sorbitanester, Sucroseester oder Gemische davon umfasst, wobei das Gemisch von Tensiden einen Gesamt-HLB-Wert im Bereich von 11 bis 13 aufweist und multilamellare Vesikel mit einer Tropfengröße von weniger als 2500 Angström und einem Doppelschicht-Abstand von unter 60 Angström produziert, und
mindestens eines aus festen, flüssigen oder gasförmigen Partikeln, suspendiert in dem strukturierten Tensidsystem.

11. Körperpflegezusammensetzung nach Anspruch 10, wobei die Zusammensetzung ferner mindestens eine hydroxylgruppenhaltige Verbindung mit mindestens zwei Hydroxylgruppen umfasst.

12. Körperpflegezusammensetzung nach Anspruch 11, wobei die hydroxylgruppenhaltige Verbindung Glycerin, ein Polyglycerin oder Gemische davon umfasst.

13. Körperpflegezusammensetzung nach den Ansprüchen 10-12, wobei das mindestens eine Tensid mit einem HLB-Wert von weniger als 10 linear ist und keine voluminöse hydrophile Kopfgruppe aufweist.

14. Körperpflegezusammensetzung nach den Ansprüchen 10-13, wobei das mindestens eine Tensid mit einem HLB-Wert von weniger als 10 eine gesättigte Alkylkette aufweist und über mindestens eine terminale Hydroxylgruppe verfügt.

## Revendications

1. Système de tensioactifs structuré aqueux, comprenant :
de l'eau et un mélange de tensioactifs présent en une quantité de 5 % à 40 % en poids du système de tensioactifs structuré, où le mélange de tensioactifs comprend :
au moins un tensioactif ayant un rapport HLB inférieur à 10, choisi parmi des esters caprylate/caprate de glycéryle et des mélanges de tels esters : et
au moins un tensioactif ayant un rapport HLB de 10 ou plus, choisi parmi un oxyde d'amine, un alkylsulfate, un alkyléthersulfate, des esters alpha-sulfométhyliques, le cholate de sodium, un alkyl-polyglucoside, des esters éthoxylés de sorbitane, des esters de saccharose, et des mélanges de ceux-ci, le mélange de tensioactifs ayant un rapport HLB global dans la plage de 11 à 13 ;
où le système de tensioactifs structuré est composé de vésicules multilamellaires ayant une taille de gouttelette inférieure à 2 500 angströms et un espacement de bicouche inférieur à 60 angströms ;
où le système de tensioactifs structuré est exempt d'électrolytes et d'épaississants polymères ; et
où le système de tensioactifs structuré est transparent en l'absence de toute matière en suspension et a des propriétés de mise en suspension.

2. Système de tensioactifs structuré aqueux selon la revendication 1, où le système de tensioactifs structuré comprend encore des composés hydroxylés comportant au moins deux groupes hydroxy.

3. Système de tensioactifs structuré aqueux selon la revendication 2, dans lequel les composés hydroxylés comprennent le glycérol, un polyglycérol ou des mélanges de ceux-ci.

4. Système de tensioactifs structuré aqueux selon les revendications 1 à 3, où le système de tensioactifs structuré comprend encore des particules de solide, liquide ou gaz en suspension de façon stable au sein du système.

5. Système de tensioactifs structuré aqueux selon les revendications 1 à 4, dans lequel le rapport dudit au moins un tensioactif ayant un rapport HLB inférieur à 10 audit au moins un tensioactif ayant un rapport HLB de 10 ou plus présents dans le système vaut de 4:1 à 1:4.

6. Système de tensioactifs structuré aqueux selon les revendications 1 à 5, dans lequel ledit au moins un tensioactif ayant un rapport HLB inférieur à 10 est linéaire et ne possède pas un groupe de tête hydrophile volumineux.

7. Système de tensioactifs structuré aqueux selon les revendications 1 à 6, dans lequel ledit au moins un tensioactif ayant un rapport HLB inférieur à 10 comporte une chaîne alkyle saturée et possède au moins un groupe hydroxy terminal.

8. Composition comprenant le système de tensioactifs structuré selon l'une quelconque des revendications 1 à 7.

9. Composition selon la revendication 8, où la composition est une composition de soins personnels, une préparation pharmaceutique, une composition pour le lavage du linge, une composition d'assouplissant pour tissus, une composition agricole ou un produit de nettoyage pour surfaces dures.

10. Composition de soins personnels, comprenant :
un système de tensioactifs structuré, comprenant de l'eau et un mélange de tensioactifs en une quantité de 5 % à 40 % en poids du système de tensioactifs structuré, où le mélange de tensioactifs comprend au moins un tensioactif ayant un rapport HLB inférieur à 10 et au moins un tensioactif ayant un rapport HLB de 10 ou plus, où ledit au moins un tensioactif ayant un rapport HLB inférieur à 10 comprend des esters caprylate/caprate de glycéryle et ledit au moins un tensioactif ayant un rapport HLB de 10 ou plus comprend un oxyde d'amine, un alkylsulfate, un alkyléthersulfate, un alkyl-polyglucoside, des esters éthoxylés de sorbitane, des esters de saccharose, ou des mélanges de ceux-ci, où le mélange de tensioactifs a un rapport HLB global dans la plage de 11 à 13 et produit des vésicules multilamellaires ayant une taille de gouttelette inférieure à 2 500 angströms et un espacement de bicouche inférieur à 60 angströms ; et
au moins une parmi des particules solides, liquides ou gazeuses mises en suspension dans le système de tensioactifs structuré.

11. Composition de soins personnels selon la revendication 10, où la composition comprend encore au moins un composé hydroxylé comportant au moins deux groupes hydroxy.

12. Composition de soins personnels selon la revendication 11, dans laquelle le composé hydroxylé comprend le glycérol, un polyglycérol ou des mélanges de ceux-ci.

13. Composition de soins personnels selon les revendications 10 à 12, dans laquelle ledit au moins un tensioactif ayant un rapport HLB inférieur à 10 est linéaire et ne possède pas un groupe de tête hydrophile volumineux.

14. Composition de soins personnels selon les revendications 10 à 13, dans laquelle ledit au moins un tensioactif ayant un rapport HLB inférieur à 10 comporte une chaîne alkyle saturée et possède au moins un groupe hydroxy terminal.
